# EUROPÄISCHE PATENTSCHRIFT

(11) **EP 0 640 588 B1**
(45) Veröffentlichungstag und Bekanntmachung des Hinweises auf die Patenterteilung: **08.09.1999**
(21) Anmeldenummer: 94113047.8
(22) Anmeldetag: 22.08.1994
(51) Int. Cl.: C07C 279/22, A61K 31/155, C07D 233/60

(54) **Ortho-substituierte Benzoylguanidine, Verfahren zu ihrer Herstellung, ihre Verwendung als Medikament oder Diagnostikum sowie sie enthaltendes Medikament**
Orthosubstituted benzoylguanidines, process for their preparation, their use as a medicament or diagnostic agent and a medicament containing them
Benzoylguanidines orthosubstituées, procédé pour leur préparation, leur utilisation comme médicament ou agent diagnostique et médicament les contenant

(30) Priorität: 27.08.1993 DE 4328869
(43) Veröffentlichungstag der Anmeldung: 01.03.1995
(73) Patentinhaber: HOECHST AKTIENGESELLSCHAFT, 65929 Frankfurt am Main (DE)
(72) Erfinder: Weichert, Andreas, Dr., D-63329 Egelsbach (DE); Lang, Hans-Jochen, Dr., D-65719 Hofheim/Ts. (DE); Kleemann, Heinz-Werner, Dr., D-61350 Bad Homburg (DE); Schwark, Jan-Robert, Dr., D-65929 Frankfurt (DE); Scholz, Wolfgang, Dr., D-65760 Eschborn (DE); Albus, Udo, Dr., D-61197 Florstadt (DE)

(56) Entgegenhaltungen:
- EP-A- 0 556 673
- EP-A- 0 556 674
- STN-INFORMATION SERVICE, FILE: REGISTRY 'RN:53497-41-9'

## Beschreibung

Die Erfindung betrifft Benzoylguanidine der Formel I 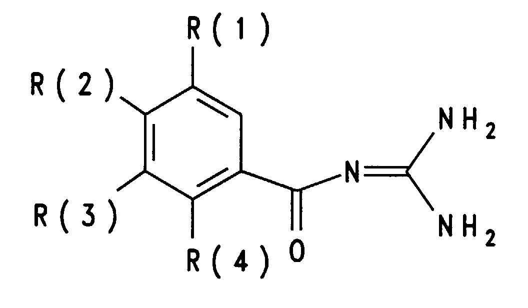 worin bedeuten:
R(1) H, F, Cl, Br, I, CN, NO₂, (C₁-C₈)-Alkyl, (C₃-C₈)-Cycloalkyl, Xₐ-(CH₂)_{b}-(CF₂)_{c}-CF₃,
   X Sauerstoff, S, NR(5),
   a Null, 1,
   b Null, 1, 2,
   c Null, 1, 2, 3,
      R(5) H, (C₁-C₄)-Alkyl, -C_{d}H_{2d}R(6),
         d Null, 1, 2, 3, 4,
         R(6) (C₃-C₈)-Cycloalkyl, Phenyl, Biphenylyl, Naphthyl,
            wobei die Aromaten nicht substituiert oder substituiert sind mit 1 bis 3 Substituenten aus der Gruppe F, Cl, CF₃, Methyl, Methoxy oder NR(7)R(8), mit R(7) und R(8) unabhängig gleich H, oder (C₁-C₄)-Alkyl,
   oder
R(1) -SR(10),-OR(10),-CR(10)R(11)R(12),
   R(10) -C_{f}H_{2f}-(C₃-C₈)-Cycloalkyl, -(C₁-C₉)-Heteroaryl oder Phenyl, wobei die aromatischen Systeme unsubstituiert oder substituiert sind mit 1 bis 3 Substituenten aus der Gruppe F, Cl, CF₃, CH₃, Methoxy, Hydroxy, Amino, Methylamino, Dimethylamino,
      f Null, 1, 2,
   R(11), R(12) unabhängig voneinander
      wie R(10) definiert oder Wasserstoff, (C₁ -C₄)-Alkyl,
   oder
R(1) Phenyl, Naphthyl, Biphenylyl, (C₁-C₉)-Heteroaryl,
   letzteres über C oder N verknüpft,
   und die unsubstituiert oder substituiert sind mit 1 bis 3 Substituenten aus der Gruppe F, Cl, CF₃, CH₃, Methoxy, Hydroxy, Amino, Methylamino oder Dimethylamino,
   oder
R(1) -SR(13), -OR(13), -NHR(13), -NR(13)R(14), -CHR(13)R(15), -C[R(15)R(16)]OH, -C≡CR(18), -C[R(19)] =CHR(18), -[CR(20)R(21)]ₖ-(CO)-[CR(22)R(23)R(24)]ₗ
   R(13), R(14) gleich oder verschieden
      -(CH₂)_{g}-(CHOH)ₕ-(CH₂)ᵢ-(CHOH)j-R(17),
         R(17) Wasserstoff, Methyl,
      -(CH₂)_{g}-O-(CH₂-CH₂O)ₕ-R(24),
         g, h, i gleich oder verschieden
            Null, 1, 2, 3, 4,
         j 1, 2, 3, 4,
   R(15), R(16) gleich oder verschieden
      Wasserstoff, (C₁ -C₆)-Alkyl oder zusammen mit dem sie tragenden Kohlenstoff-Atom ein (C₃-C₈)-Cycloalkyl,
   R(18) Phenyl,
      das unsubstituiert oder substituiert ist mit 1 bis 3 Substituenten aus der Gruppe F, Cl, CF₃, Methyl, Methoxy oder NR(25)R(26) mit R(25) und R(26) gleich H oder (C₁ -C₄)-Alkyl, oder
   R(18) (C₁-C₉)-Heteroaryl,
      das unsubstituiert oder wie Phenyl substituiert ist, oder
   R(18) (C₁-C₆)-Alkyl,
      das unsubstituiert oder mit 1 bis 3 OH substituiert ist, oder
   R(18) (C₃-C₈)-Cycloalkyl, und
   R(19), R(20), R(21), R(22) und R(23)
      Wasserstoff, Methyl,
         k Null, 1, 2, 3, 4,
         l Null, 1, 2, 3, 4,
   R(24) H, (C₁-C₆)-Alkyl, (C₃-C₈)-Cycloalkyl, -CₘH₂ₘ-R(18),
      m 1, 2, 3, 4,
R(2) und R(3) unabhängig voneinander wie R(1) definiert, und
R(4) (C₁ -C₃)-Alkyl, F, Cl, Br, I, CN, -(CH₂)ₙ-(CF₂)ₒ-CF₃,
   n Null, 1,
   o Null, 1, 2,
   wobei jedoch die Verbindung o-Chlorbenzoylguanidin-hydrochlorid ausgeschlossen ist;
   sowie deren pharmazeutisch verträgliche Salze.

Bevorzugt sind Verbindungen der Formel I, in welcher bedeuten:
R(1) H, F, Cl, Br, CN, NO₂, (C₁ -C₈)-Alkyl, (C₃-C₈)-Cycloalkyl, Xₐ-(CF₂)_{c}-CF₃,
   X Sauerstoff, S,
   a Null, 1,
   c Null, 1, 2, 3,
   oder
R(1) -SR(10),-OR(10),
   R(10) -C_{f}H_{2f}-(C₃-C₈)-Cycloalkyl, -(C₁-C₉)-Heteroaryl oder Phenyl, wobei die aromatischen Systeme unsubstituiert oder substituiert sind mit 1 bis 3 Substituenten aus der Gruppe F, Cl, CF₃, CH₃, Methoxy, Hydroxy, Amino, Methylamino, Dimethylamino,
      f Null, 1,
   oder
R(1) Phenyl, Naphthyl, Biphenylyl, (C₁-C₉)-Heteroaryl,
   letzteres über C oder N verknüpft,
   und die unsubstituiert oder substituiert sind mit 1 bis 3 Substituenten aus der Gruppe F, Cl, CF₃, CH₃, Methoxy, Hydroxy, Amino, Methylamino oder Dimethylamino;
   oder
R(I) -SR(13), -OR(13), -NHR(13), -NR(13)R(14), -C≡CR(18), - C[(R(19)] = CHR(18),
   R(13), R(14) gleich oder verschieden
      -(CH₂)_{g}-(CHOH)ₕ-(CH₂)ᵢ-(CHOH)ⱼ-R(17),
         R(17) Wasserstoff, Methyl;
      -(CH₂)_{g}-O(CH₂-CH₂O)ₕ-R(24),
         g, h, i gleich oder verschieden
            Null, 1, 2,
         j 1, 2,
   R(18) Phenyl,
      das unsubstituiert oder substituiert ist mit 1 bis 3 Substituenten aus der Gruppe F, Cl, CF₃, Methyl, Methoxy oder NR(25)R(26) mit R(25) und R(26) gleich H oder (C₁-C₄)-Alkyl, oder
   R(18) (C₁-C₉)-Heteroaryl,
      das unsubstituiert oder wie Phenyl substituiert ist, oder
   R(18) (C₁ -C₆)-Alkyl,
      das unsubstituiert oder mit 1 bis 3 OH substituiert ist, oder
   R(18) (C₃-C₈)-Cycloalkyl, und
   R(19) Wasserstoff, Methyl,
R(2) und R(3) unabhängig voneinander wie R(1) definiert, und
R(4) (C₁ -C₂)-Alkyl, F, Cl, Br, CN, -(CH₂)ₙ-(CF₂)ₒ-CF3,
   n Null, 1,
   o Null, 1,
   sowie deren pharmazeutisch verträgliche Salze.

Ganz besonders bevorzugt sind Verbindungen der Formel I, in welcher bedeuten:
R(1) H, F, Cl, (C₁-C₈)-Alkyl, (C₅-C₆)-Cycloalkyl, Xₐ-(CF₂)_{c}-CF₃,
   X Sauerstoff,
   a Null, 1,
   c Null, 1,
   oder
R(1) -SR(10), -OR(10),
   R(10) -(C₄-C₆)-Cycloalkyl, Chinolyl, Isochinolyl, Pyridyl, Phenyl,
      das unsubstituiert oder substituiert ist mit 1 bis 3 Substituenten aus der Gruppe F, Cl, CF₃, CH₃, Methoxy, Hydroxy, Amino, Methylamino, Dimethylamino,
   oder
R(1) Phenyl, Chinolyl, Isochinolyl, Pyridyl, Imidazolyl,
   über C oder N gebunden,
   die unsubstituiert oder substituiert sind mit 1 bis 3 Substituenten aus der Gruppe F, Cl, CF₃, CH₃, Methoxy, Hydroxy, Amino, Methylamino oder Dimethylamino,
   oder
R(1) -C≡CR(18),
   R(18) Phenyl, (C₅-C₆)-Cycloalkyl,
R(2) und R(3) unabhängig voneinander wie R(1) definiert, und
R(4) Methyl, F, Cl, CF₃,
   sowie deren pharmazeutisch verträgliche Salze.

Unter (C₁-C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als (C₁-C₉)-Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(4) ein oder mehrere Asymmetriezentren, so können diese sowohl S als auch R konfiguriert sein. Die Verbindungen können als optische Isomere, als Diastereomere, als Racemate oder als Gemische derselben vorliegen. Alkylreste können sowohl geradkettig als auch verzweigt vorliegen.

Die Erfindung betrifft weiterhin ein Verfahren zur Herstellung der Verbindung I, dadurch gekennzeichnet, daß man eine Verbindung der Formel II 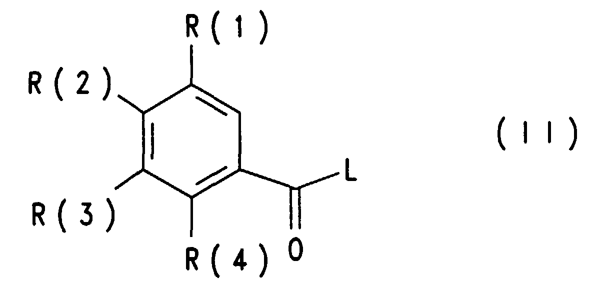 worin R(1) bis R(4) die in Anspruch 1 angegebene Bedeutung besitzen und L für eine leicht nucleophil substituierbare leaving group steht, mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 bis 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluroni um-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 3-, 4-, 5- und 6-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. - zinkverbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 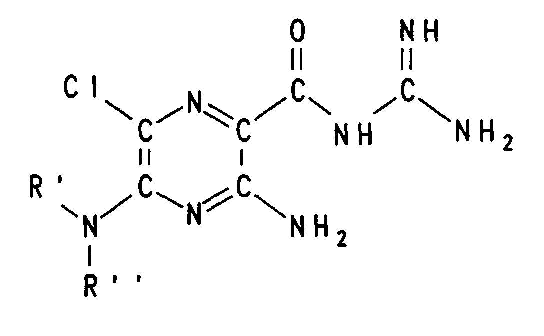
Amilorid: R', R'' = H
Dimethylamilorid: R', R'' = CH₃
Ethylisopropylamilorid: R' = C₂H₅, R'' = CH(CH₃)₂

Darüber hinaus sind Untersuchungen bekannt geworden, die auf antiarrhythmische Eigenschaften von Amilorid hinweisen (Circulation 79, 1257 bis 1263 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl. 1): 167 (1988) (book of abstracts)). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der EP-A-416 499 und in der EP-A-556 674 werden Benzoylguanidine beschrieben, die jedoch am aromatischen Kern keine ortho-ständigen Substituenten tragen.
Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch schäme ausgelösten Schäden oder dadurch primär oder sekundär induzierten Krankheiten verwendet werden. Dies betrifft ihre Verwendung als Arzneimittel für operative Eingriffe, z. B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z. B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel l und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- RT: Raumtemperatur
- EE: Ethylacetat (EtOAc)
- Smp: Schmelzpunkt
- THF: Tetrahydrofuran
- eq.: Äquivalent
- ZNS: Zentralnervensystem

### Experimenteller Teil

### Allgemeine Vorschrift zur Herstellung von Benzoyl-guanidinen (I) Variante A: aus Benzoesäuren (II, L = OH)

1.0 eq. des Benzoesäurederivates der Formel II löst bzw. suspendiert man in wasserfreiem THF (5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotavapor) ab, versetzt mit Wasser, stellt mit 2 N HCl auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Sieden erhitzt (typische Reaktionszeit 2 bis 5 Stunden). Das Lösungsmittel wird unter vermindertem Druck (Rotavapor) abdestilliert, der Rückstand wird in EE aufgenommen und 3 x mit NaHCO₃-Lösung gewaschen. Es wird über Na₂SO₄ getrocknet, das Lösungsmittel im Vakuum abdestilliert, und der Rückstand wird an Kieselgel mit einem geeigneten Laufmittel, z. B. EE/MeOH
5 : 1 chromatographiert.
(Salzbildung vergleiche Variante A)

### Beispiel 1:

2,3-Dichloro-benzoylguanidin-hydrochlorid: Farblose Kristalle, Smp. 232°C
aus 2,3-Dichlorbenzoesäure nach Variante A

### Beispiel 2:

2-Fluoro-3-trifluoromethyl-benzoylguanidin-hydrochlorid: Farblose Kristalle, glasartig
aus 2-Fluoro-3-trifluoromethyl-benzoesäure nach Variante B

### Beispiel 3:

2,4-Dichloro-benzoylguanidin-hydrochlorid: Farblose Kristalle, Smp. 242°C
aus 2,4-Dichlorbenzoesäure nach Variante A

### Beispiel 4:

2-Fluoro-4-trifluoromethyl-benzoylguanidin-hydrochlorid: Farblose Kristalle, Smp. 143 bis 145°C
aus 2-Fluoro-4-trifluoromethyl-benzoesäure nach Variante B

### Beispiel 5:

2,5-Dimethyl-benzoylguanidin-hydrochlorid: Farblose Kristalle, Smp. 247°C
aus 2,5-Dimethyl-benzoesäure nach Variante A.

### Beispiel 6:

2,5-Dichloro-benzoylguanidin-hydrochlorid: Farblose Kristalle, Smp. 209°C
aus 2,5-Dichloro-benzoesäure nach Variante A.

### Beispiel 7:

2,5-Bis(trifluormethyl)-benzoylguanidin-hydrochlorid: Farblose Kristalle, Smp. 240 bis 242°C
aus 2,5-Bis(trifluormethyl)-benzoesäure nach Variante A.

### Beispiel 8:

2-Fluoro-5-trifluoromethyl-benzoylguanidin-hydrochlorid: Farblose Kristalle, Smp. 168 bis 170°C
aus 2-Fluoro-5-trifluoromethyl-benzoesäure nach Variante A.

### Beispiel 9:

2-Chloro-5-trifluoromethyl-benzoylguanidin-hydrochlorid: Farblose Kristalle, Smp. 209 - 11°C
aus 2-Chloro-5-trifluoromethyl-benzoesäure nach Variante A.

### Beispiel 10:

2-Fluoro-5-iodo-3-trifluoromethyl-benzoylguanidin-hydrochlorid: Farblose Kristalle, Smp. 197-200°C

### Syntheseweg:

a) 2-Fluoro-5-iodo-3-trifluoromethyl-benzoesäurenitril aus 2-Fluoro-3-trifluoromethyl-benzonitril durch Reaktion mit 1 Äquivalent N-Iod-Succinimid in 5 Äquivalenten Trifluormethansulfonsäure bei RT für 24 h, farblose Kristalle, Smp. 65 - 67°C
b) 2-Fluoro-5-iodo-3-trifluoromethyl-benzoesäure aus a) durch Erhitzen eines Gemisches aus konz. Salzsäure in Eisessig, farblose Kristalle, Smp. 136 - 38°C
c) 2-Fluoro-5-iodo-3-trifluoromethyl-benzoylguanidin-hydrochlorid aus b) nach Variante A

### Beispiel 11:

2,4-Difluoro-benzoylguanidin-hydrochlorid: Farblose Kristalle, Smp. 170 - 72°C
aus 2,4-Difluoro-benzoesäure nach Variante A.

### Beispiel 12:

4-Fluoro-2-trifluoromethyl-benzoylguanidin-hydrochlorid: Farblose Kristalle, Smp. 155 - 57°C
aus 4-Fluoro-2-trifluoromethyl-benzoesäure nach Variante A.

### Beispiel 13:

4-Imidazolyl-2-trifluoromethyl-benzoylguanidin-hydrochlorid: Farblose Kristalle, amorph
aus 4-Imidazolyl-2-trifluoromethyl-benzoesäure nach Variante A.

### Beispiel 14:

2-Trifluoromethyl-benzoylguanidin-hydrochlorid: Farblose Kristalle, Smp. 225°C
aus 2-Trifluoromethyl-benzoesäure nach Variante A.

### Beispiel 15:

2,3-Dichloro-4-methoxy-benzoylguanidin-hydrochlorid: Farblose Kristalle, Smp. 227°C
aus 2,3-Dichloro-4-methoxy-benzoesäure nach Variante A.

### Beispiel 16:

2-Chloro-5-methyl-benzoylguanidin-hydrochlorid: Farblose Kristalle, Smp. 134°C
aus 2-Chloro-5-methyl-benzoesäure nach Variante A.

### Pharmakologische Daten:

### lnhibition des Na⁺/H⁺-Exchangers von Kaninchenerythrocyten

Weiße Neuseeland-Kaninchen (Ivanovas) erhielten eine Standard-Diät mit 2 % Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺/H⁺-Exchangers:

| Beispiel | IC₅₀ (mol/l) |
|---|---|
| 1 | größer als 10⁻⁶ |
| 2 | 2 x 10⁻⁶ |
| 3 | 8 x 10⁻⁶ |
| 4 | 3 x 10⁻⁶ |
| 5 | 2 x 10⁻⁶ |
| 6 | 1,5 x 10⁻⁶ |
| 7 | 0,16 x 10⁻⁶ |
| 8 | 0,7 x 10⁻⁶ |
| 9 | 0,16 x 10⁻⁶ |
| 10 | 0,09 x 10⁻⁶ |

## Patentansprüche

1. Benzoylguanidine der Formel I 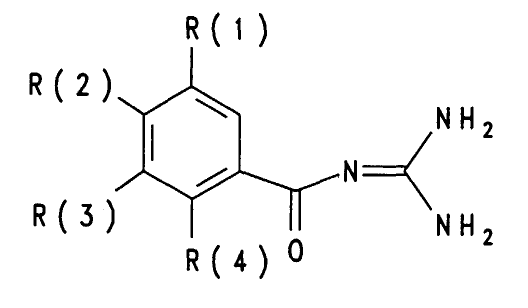 worin bedeuten:
R(1) H, F, Cl, Br, I, CN, NO₂, (C₁-C₈)-Alkyl, (C₃-C₈)-Cycloalkyl, Xₐ-(CH₂)_{b}-(CF₂)_{c}-CF₃,
X Sauerstoff, S, NR(5),
a Null, 1,
b Null, 1, 2,
c Null, 1, 2, 3,
R(5) H, (C₁-C₄)-Alkyl,-C_{d}H_{2d}R(6),
d Null, 1, 2, 3, 4,
R(6) (C₃-C₈)-Cycloalkyl, Phenyl, Biphenylyl, Naphthyl,
wobei die Aromaten nicht substituiert oder substituiert sind mit 1 bis 3 Substituenten aus der Gruppe F, Cl, CF₃, Methyl, Methoxy oder NR(7)R(8), mit R(7) und R(8) unabhängig gleich H, oder (C₁-C₄)-Alkyl,
oder
R(1) -SR(10), -OR(10), -CR(10)R(11)R(12),
R(10) -C_{f}H_{2f}-(C₃-C₈)-Cycloalkyl, -(C₁-C₉)-Heteroaryl oder Phenyl, wobei die aromatischen Systeme unsubstituiert oder substituiert sind mit 1 bis 3 Substituenten aus der Gruppe F, Cl, CF₃, CH₃, Methoxy, Hydroxy, Amino, Methylamino, Dimethylamino,
f Null, 1, 2,
R(11), R(12) unabhängig voneinander
wie R(10) definiert oder Wasserstoff, (C₁-C₄)-Alkyl,
oder
R(1) Phenyl, Naphthyl, Biphenylyl, (C₁-C₉)-Heteroaryl,
letzteres über 6 oder N verknüpft,
und die unsubstituiert oder substituiert sind mit 1 bis 3 Substituenten aus der Gruppe F, Cl, CF₃, CH₃, Methoxy, Hydroxy, Amino, Methylamino oder Dimethylamino,
oder
R(1) -SR(13),-OR(13), -NHR(13), -NR(13)R(14), -CHR(13)R(15), -C[R(15)R(16)]OH, -C≡CR(18), -C[R(19)] = CHR(18), -[CR(20)R(21)]ₖ-(CO)-[CR(22)R(23)R(24)]ₗ
R(13), R(14) gleich oder verschieden
-(CH₂)g-(CHOH)ₕ-(CH₂)ᵢ-(CHOH)ⱼ-R(17),
R(17) Wasserstoff, Methyl,
-(CH₂)_{g}-O-(CH₂-CH₂O)ₕ-R(24),
g, h, i gleich oder verschieden
Null, 1, 2, 3, 4,
j 1, 2, 3, 4,
R(15), R(16) gleich oder verschieden
Wasserstoff, (C₁-C₆)-Alkyl oder zusammen mit dem sie tragenden Kohlenstoff-Atom ein (C₃-C₈)-Cycloalkyl,
R(18) Phenyl,
das unsubstituiert oder substituiert ist mit 1 bis 3 Substituenten aus der Gruppe F, Cl, CF₃, Methyl, Methoxy oder NR(25)R(26) mit R(25) und R(26) gleich H oder (C₁-C₄)-Alkyl, oder
R(18) (C₁-C₉)-Heteroaryl,
das unsubstituiert oder wie Phenyl substituiert ist, oder
R(18) (C₁-C₆)-Alkyl,
das unsubstituiert oder mit 1 bis 3 OH substituiert ist, oder
R(18) (C₃-C₈)-Cycloalkyl, und
R(19), R(20), R(21), R(22) und R(23)
Wasserstoff, Methyl,
k Null, 1, 2, 3, 4,
l Null, 1, 2, 3, 4,
R(24) H, (C₁-C₆)-Alkyl, (C₃-C₈)-Cycloalkyl, -CₘH₂ₘ-R(18),
m 1, 2, 3, 4,
R(2) und R(3) unabhängig voneinander wie R(1) definiert, und
R(4) (C₁-C₃)-Alkyl, F, Cl, Br, I, CN, -(CH₂)ₙ-(CF₂)ₒ-CF₃,
n Null, 1,
o Null, 1, 2,
wobei jedoch die Verbindung o-Chlorbenzoylguanidin-hydrochlorid ausgeschlossen ist;
sowie deren pharmazeutisch verträgliche Salze.

2. Verbindungen der Formel I nach Anspruch 1, dadurch gekennzeichnet, daß darin bedeuten:
R(1) H, F, Cl, Br, CN, NO₂, (C₁ -C₈)-Alkyl, (C₃-C₈)-Cycloalkyl, Xₐ-(CF₂)_{c}-CF₃,
X Sauerstoff, S,
a Null, 1,
c Null, 1, 2, 3,
oder
R(1) -SR(10),-OR(10),
R(10) -C_{f}H_{2f}-(C₃-6₈)-Cycloalkyl, -(C₁-C₉)-Heteroaryl oder Phenyl, wobei die aromatischen Systeme unsubstituiert oder substituiert sind mit 1 bis 3 Substituenten aus der Gruppe F, Cl, CF₃, CH₃, Methoxy, Hydroxy, Amino, Methylamino, Dimethylamino,
f Null, 1,
oder
R(1) Phenyl, Naphthyl, Biphenylyl, (C₁-C₉)-Heteroaryl,
letzteres über C oder N verknüpft,
und die unsubstituiert oder substituiert sind mit 1 bis 3 Substituenten aus der Gruppe F, Cl, CF₃, CH₃, Methoxy, Hydroxy, Amino, Methylamino oder Dimethylamino,
oder
R(1) -SR(13), -OR(13), -NHR(13), -NR(13)R(14), -C≡CR(18), - C[R(19)] = CHR(18),
R(13), R(14) gleich oder verschieden
-(CH₂)_{g}-(CHOH)ₕ-(CH₂)ᵢ-(CHOH)ⱼ-R(17),
R(17) Wasserstoff, Methyl,
-(CH₂)_{g}-O-(CH₂-CH₂O)ₕ-R(24),
g, h, i gleich oder verschieden
Null, 1, 2,
j 1, 2,
R(18) Phenyl,
das unsubstituiert oder substituiert ist mit 1 bis 3 Substituenten aus der Gruppe F, Cl, CF₃, Methyl, Methoxy oder NR(25)R(26) mit R(25) und R(26) gleich H oder (C₁-C₄)-Alkyl, oder
R(18) (C₁-C₉)-Heteroaryl,
das unsubstituiert oder wie Phenyl substituiert ist, oder
R(18) (C₁-C₆)-Alkyl,
das unsubstituiert oder mit 1 bis 3 OH substituiert ist, oder
R(18) (C₃-C₈)-Cycloalkyl, und
R(19) Wasserstoff, Methyl,
R(2) und R(3) unabhängig voneinander wie R(1) definiert, und
R(4) (C₁-C₂)-Alkyl, F, Cl, Br, CN, -(CH₂)ₙ-(CF₂)ₒ-CF₃,
n Null, 1,
o Null, 1,
sowie deren pharmazeutisch verträgliche Salze.

3. Verbindungen der Formel I nach Anspruch 1, dadurch gekennzeichnet, daß darin bedeuten:
R(1) H, F, Cl, (C₁ -C₈)-Alkyl, (C₅-C₆)-Cycloalkyl, Xₐ-(CF₂)_{c}-CF₃,
X Sauerstoff,
a Null, 1,
c Null, 1,
oder
R(1) -SR(10), -OR(10),
R(10) -(C₄-C₆)-Cycloalkyl, Chinolyl, Isochinolyl, Pyridyl, Phenyl,
das unsubstituiert oder substituiert ist mit 1 bis 3 Substituenten aus der Gruppe F, Cl, CF₃, CH₃, Methoxy, Hydroxy, Amino, Methylamino, Dimethylamino,
oder
R(1) Phenyl, Chinolyl, Isochinolyl, Pyridyl, Imidazolyl,
über C oder N gebunden,
die unsubstituiert oder substituiert sind mit 1 bis 3 Substituenten aus der Gruppe F, Cl, CF₃, CH₃, Methoxy, Hydroxy, Amino, Methylamino oder Dimethylamino,
oder
R(1) -C≡CR(18),
R(18) Phenyl, (C₅-C₆)-Cycloalkyl,
R(2) und R(3) unabhängig voneinander wie R(1) definiert, und
R(4) Methyl, F, Cl, CF₃,
sowie deren pharmazeutisch verträgliche Salze.

4. Verfahren zur Herstellung einer Verbindung I nach Anspruch 1, dadurch gekennzeichnet, daß man
eine Verbindung der Formel II 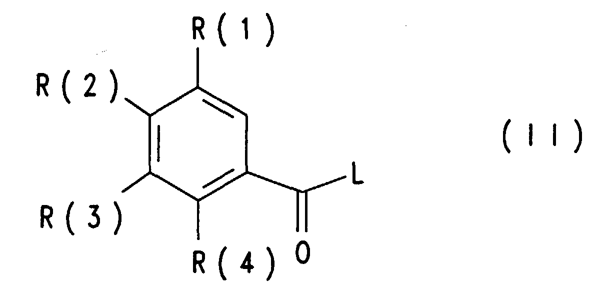 worin R(1) bis R(4) die in Anspruch 1 angegebene Bedeutung besitzen und L für eine leicht nucleophil substituierbare leaving group steht,
mit Guanidin umsetzt.

5. Verwendung einer Verbindung I nach Anspruch 1 zur Herstellung eines Medikaments zu Behandlung von Arrhythmien.

6. Verwendung einer Verbindung l nach Anspruch 1 zur Herstellung eines Medikaments zur Behandlung oder Prophylaxe des Herzinfarktes.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung l nach Anspruch 1 zur Herstellung eines Medikaments zur Behandlung oder Prophylaxe von ischämischen Zuständen peripherer Organe und Gliedmaßen.

11. Verwendung einer Verbindung l nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung von Krankheiten, bei denen die Zellproliferation eine primäre oder sekundäre Ursache darstellt.

15. Heilmittel, gekennzeichnet durch einen wirksamen Gehalt einer Verbindung I nach Anspruch 1.

## Claims

1. A benzoylguanidine of the formula I 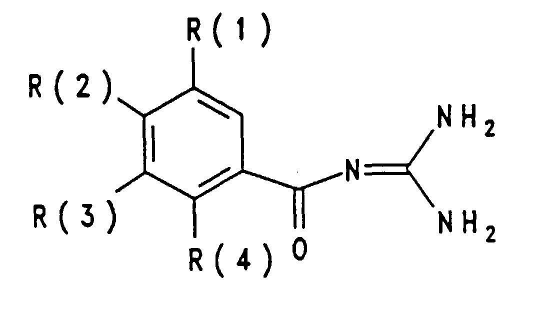 in which:
R(1) is H, F, Cl, Br, I, CN, NO₂, (C₁-C₈)-alkyl, (C₃-C₈)-cycloalkyl or Xₐ-(CH₂)_{b}-(CF₂)_{c}-CF₃,
X is oxygen, S or NR(5),
a is zero or 1,
b is zero, 1 or 2,
c is zero, 1, 2 or 3,
R(5) is H, (C₁-C₄)-alkyl or -C_{d}H_{2d}R(6),
d is zero, 1, 2, 3 or 4,
R(6) is (C₃-C₈)-cycloalkyl, phenyl, biphenylyl or naphthyl,
where the aromatic radicals are not substituted or are substituted by 1 to 3 substituents selects from the grow comprising F, Cl, CF₃, methyl, methoxy or NR(7)R(8), with R(7) and R(8) being, independently, H or (C₁-C₄)-alkyl,
or
R(1) is -SR(10), -OR(10) or -CR(10)R(11)R(12),
R(10) is -C_{f}H_{2f}-(C₃-C₈)-cycloalkyl, -(C₁-C₉)-heteroaryl or phenyl, where the aromatic systems are unsubstituted or are substituted by 1 to 3 substituents selected from the group comprising F, Cl, CF₃, CH₃, methoxy, hydroxyl, amino, methylamino or dimethylamino,
f is zero, 1 or 2,
R(11) and (R12), independently of each other,
are defined like R(10) or are hydrogen or (C₁-C₄)-alkyl,
or
R(1) is phenyl, naphthyl, biphenylyl or (C₁-C₉)-heteroaryl, the latter linked via C or N,
and which are unsubstituted or are substituted by 1 to 3 substituents selected from the group comprising F, Cl, CF₃, CH₃, methoxy, hydroxyl, amino, methylamino or dimethylamino,
or
(R1) is -SR(13), -OR(13), -NHR(13), -NR(13)R(14), -CHR(13)R(15), -C[R(15)R(16)]OH, -C≡CR(18), -C[R(19)]=CHR(18), -[CR(20)R(21)]ₖ-(CO)-[CR(22)R(23)R(24)]ₗ
R(13) and R(14), identically or differently, are
-(CH₂)_{g}-(CHOH)ₕ-(CH₂)ᵢ-(CHOH)ⱼ-R(17),
R(17) is hydrogen or methyl, or
-(CH₂)_{g}-O-(CH₂CH₂O)ₕ-R(24),
g, h and i, identically or differently, are
zero, 1, 2, 3 or 4,
j is 1, 2, 3 or 4,
R(15) and R(16), identically or differently, are
hydrogen, (C₁-C₆) -alkyl or, together with the carbon atom carrying them, a (C₃-C₈)-cycloalkyl,
R(18) is phenyl,
which is unsubstituted or is substituted by 1 to 3 substituents selected from the group comprising F, Cl, CF₃, methyl, methoxy or NR(25)R(26) with R(25) and R(26) being H or (C₁-C₄)-alkyl, or
R(18) is (C₁-C₉)-heteroaryl,
which is unsubstituted or is substituted like phenyl, or
R(18) is (C₁-C₆)-alkyl,
which is unsubstituted or is substituted by 1 to 3 OH, or
R(18) is (C₃-C₈)-cycloalkyl, and
R(19), R(20), R(21), R(22) and R(23)
are hydrogen or methyl,
k is zero, 1, 2, 3 or 4,
1 is zero, 1, 2, 3 or 4,
R(24) is H, (C₁-C₆)-alkyl, (C₃-C₈)-cycloalkyl or -CₘH₂ₘ-R(18),
m is 1, 2, 3 or 4,
R(2) and R(3), independently of each other, are defined like R(1), and
R(4) is (C₁-C₃)-alkyl, F, Cl, Br, I, CN or -(CH₂)ₙ-(CF₂)ₒ-CF₃,
n is zero or 1,
o is zero, 1 or 2,
the compound o-chlorobenzoylguanidine hydrochloride, however, being excluded;
as well as pharmaceutically tolerated salts thereof.

2. The compound of the formula I as claimed in claim 1, wherein:
R(1) is H, F, Cl, Br, CN, NO₂, (C₁-C₈)-alkyl, (C₃-C₈)-cycloalkyl or Xₐ-(CF₂)_{c}-CF₃,
X is oxygen or S,
a is zero or 1,
c is zero, 1, 2 or 3,
or
R(1) is -SR(10) or -OR(10),
R(10) is -C_{f}H_{2f}-(C₃-C₈)-cycloalkyl, -(C₁-C₉)-heteroaryl or phenyl, where the aromatic systems are unsubstituted or are substituted by 1 to 3 substituents selected from the group comprising F, Cl, CF₃, CH₃, methoxy, hydroxyl, amino, methylamino or dimethylamino,
f is zero or 1,
or
R(1) is phenyl, naphthyl, biphenylyl or (C₁-C₉)-heteroaryl, the latter linked via C or N,
and which are unsubstituted or are substituted by 1 to 3 substituents selected from the group comprising F, Cl, CF₃, CH₃, methoxy, hydroxyl, amino, methylamino or dimethylamino,
or
(R1) is -SR(13), -OR(13), -NHR(13), -NR(13)R(14), -C≡CR(18) or -C[R(19)]=CHR(18),
R(13) and R(14), identically or differently, are
-(CH₂)_{g}-(CHOH)ₕ-(CH₂)ᵢ-(CHOH)₃-R(17),
R(17) is hydrogen or methyl, or
-(CH₂)_{g}-O-(CH₂-CH₂O)ₕ-R(24),
g, h and i, identically or differently, are
zero, 1 or 2,
j is 1 or 2,
R(18) is phenyl,
which is unsubstituted or is substituted by 1 to 3 substituents selected from the group comprising F, Cl, CF₃, methyl, methoxy or NR(25)R(26) with R(25) and R(26) being H or (C₁-C₄)-alkyl, or
R(18) is (C₁-C₉)-heteroaryl,
which is unsubstituted or is substituted like phenyl, or
R(18) is (C₁-C₆)-alkyl,
which is unsubstituted or is substituted by 1 to 3 OH, or
R(18) is (C₃-C₈)-cycloalkyl, and
R(19) is hydrogen or methyl,
R(2) and R(3), independently of each other, are defined like R(1), and
R(4) is (C₁-C₂)-alkyl, F, Cl, Br, CN or -(CH₂)ₙ-(CF₂)ₒ--CF₃,
n is zero or 1,
o is zero or 1,
as well as pharmaceutically tolerated salts thereof.

3. The compound of the formula I as claimed in claim 1, wherein:
R(1) is H, F, Cl, (C₁-C₈)-alkyl, (C₅-C₆)-cycloalkyl or Xₐ-(CF₂)_{c}-CF₃,
X is oxygen,
a is zero or 1,
c is zero or 1,
or
R(1) is -SR(10) or -OR(10),
R(10) is -(C₄-C₆)-cycloalkyl, quinolyl, isoquinolyl, pyridyl or phenyl,
which is unsubstituted or is substituted by 1 to 3 substituents selected from the group comprising F, Cl, CF₃, CH₃, methoxy, hydroxyl, amino, methylamino or dimethylamino,
or
R(1) is phenyl, quinolyl, isoquinolyl, pyridyl or imidazolyl,
bound via C or N,
which are unsubstituted or are substituted by 1 to 3 substituents selected from the group comprising F, Cl, CF₃, CH₃, methoxy, hydroxyl, amino, methylamino or dimethylamino,
or
R(1) is -C≡CR(18),
R(18) is phenyl or (C₅-C₆)-cycloalkyl,
R(2) and R(3), independently of each other, are defined like R(1), and
R(4) is methyl, F, Cl or CF₃,
as well as pharmaceutically tolerated salts thereof.

4. A process for preparing a compound I as claimed in claim 1, wherein
a compound of the formula II 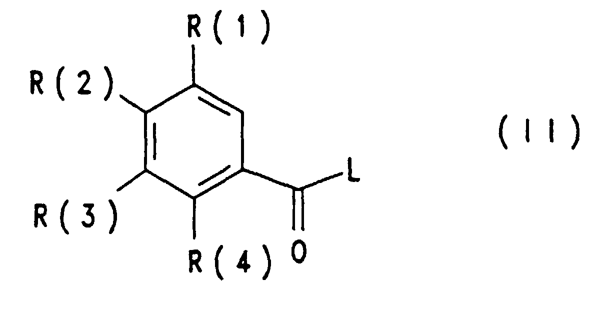 in which R(1) to R(4) have the meaning given in claim 1 and L is a leaving group which can readily be substituted nucleophilically,
is reacted with guanidine.

5. Use of a compound I as claimed in claim 1 for preparing a medicament for treating arrhythmias.

6. Use of a compound I as claimed in claim 1 for preparing a medicament for the treatment or prophylaxis of cardiac infarction.

7. Use of a compound I as claimed in claim 1 for preparing a medicament for the treatment or prophylaxis of angina pectoris.

8. Use of a compound I as claimed in claim 1 for preparing a medicament for the treatment or prophylaxis of ischemic conditions of the heart.

9. Use of a compound I as claimed in claim 1 for preparing a medicament for the treatment or prophylaxis of ischemic conditions of the peripheral and central nervous system and of stroke.

10. Use of a compound I as claimed in claim 1 for preparing a medicament for the treatment or prophylaxis of ischemic conditions of peripheral organs and limbs.

11. Use of a compound I as claimed in claim 1 for preparing a medicament for the treatment of conditions of shock.

12. Use of a compound I as claimed in claim 1 for preparing a medicament for employment in surgical operations and organ transplantations.

13. Use of a compound I as claimed in claim 1 for preparing a medicament for the preservation and storage of transplants for surgical measures.

14. Use of a compound I as claimed in claim 1 for preparing a medicament for the treatment of diseases in which cell proliferation represents a primary or secondary cause.

15. A medicine which comprises an effective content of a compound I as claimed in claim 1.

## Revendications

1. Benzoylguanidines de formule I 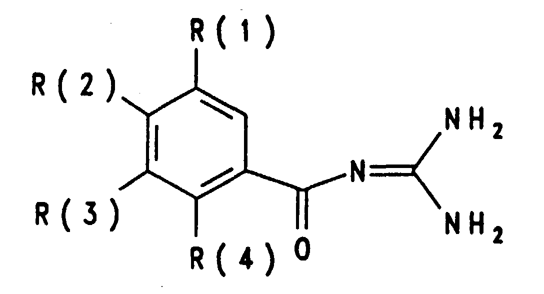 dans laquelle
R(1) représente H, F, Cl, Br, I, CN, NO₂, un groupe alkyle en C₁-C₈, cycloalkyle en C₃-C₈, Xₐ-(CH₂)_{b}-(CF₂)_{c}-CF₃,
X représente un atome d'oxygène ou de soufre ou NR(5),
a est égal à zéro ou 1,
b est égal à zéro, 1 ou 2,
c est égal à zéro, 1, 2 ou 3,
R(5) représente H ou un groupe alkyle en C₁-C₄, -C_{d}-H_{2d}R(6),
d est égal à zéro, 1, 2, 3 ou 4,
R(6) représente un groupe cycloalkyle en C₃-C₈, phényle, biphénylyle ou naphtyle,
les radicaux aromatiques n'étant pas substitués ou portant 1 à 3 substituants choisis dans l'ensemble constitué par F, Cl, CF₃, les groupes méthyle, méthoxy et NR(7)R(8),
R(7) et R(8) représentant indépendamment H ou un groupe alkyle en C₁-C₄;
ou
R(1) représente -SR(10), -OR(10), -CR(10)R(11)R(12),
R(10) représente un groupe
-C_{f}H_{2f}-cycloalkyle(C₃-C₈), hétéroaryle en C₁-C₉ ou phényle, les systèmes aromatiques n'étant pas substitués ou portant 1 à 3 substituants choisis dans l'ensemble constitué par F, Cl, CF₃, CH₃, les groupes méthoxy, hydroxyle, amino, méthylamino, diméthylamino,
f étant égal à zéro, 1 ou 2,
R(11), R(12), indépendamment l'un de l'autre,
sont définis comme R(10) ou représentent un atome d'hydrogène ou un groupe alkyle en C₁-C₄,
ou
R(1) représente un groupe phényle, naphtyle, biphénylyle, hétéroaryle en C₁-C₉,
ce dernier étant relié par C ou N,
et qui ne sont pas substitués ou portent 1 à 3 substituants choisis dans l'ensemble constitué par F, Cl, CF₃, CH₃, les groupes méthoxy, hydroxyle, amino, méthylamino et diméthylamino,
ou
(R1) représente -SR(13), -OR(13), -NHR(13), -NR(13)R(14), -CHR(13)R(15), -C[R(15)R(16)]OH, -C≡CR(18), -C[R(19)]=CHR(18), -[CR(20)R(21)]ₖ-(CO)-[CR(22)R(23)R(24)]ₗ
R(13), R(14) sont identiques ou différents et représentent un groupe
-(CH₂)_{g}-(CHOH)ₕ-(CH₂)ᵢ-(CHOH)ⱼ-R(17),
R(17) étant un atome d'hydrogène ou le groupe méthyle,
-(CH₂)_{g}-O-(CH₂-CH₂O)ₕ-R(24),
g, h, i étant identiques ou différents
et représentant zéro, 1, 2, 3, 4,
j représentant 1, 2, 3, 4,
R(15), R(16) sont identiques ou différents, et représentent
un atome d'hydrogène ou un groupe alkyle en C₁-C₆, ou forment ensemble, avec l'atome de carbone qui les porte, un groupe cycloalkyle en C₃-C₈,
R(18) représente un groupe phényle
qui est non substitué ou porte 1 à 3 substituants choisis dans l'ensemble constitué par F, Cl, CF₃, les groupes méthyle, méthoxy ou NR(25)R(26), R(25) et R(26) représentant H ou un groupe alkyle en C₁-C₄, ou
R(18) représente un groupe hétéroaryle en C₁-C₉,
qui est non substitué ou substitué comme le groupe phényle, ou
R(18) représente un groupe alkyle en C₁ -C₆,
qui n'est pas substitué ou porte 1 à 3 groupes OH, ou
R(18) représente un groupe cycloalkyle en C₃-C₈, et
R(19), R(20), R(21), R(22) et R(23) représentent un atome d'hydrogène ou le groupe méthyle,
k représente zéro, 1, 2, 3, 4,
l représente zéro, 1, 2, 3, 4,
R(24) représente H ou un groupe alkyle en C₁-C₆, cycloalkyle en C₃-C₈, -CₘH₂ₘ-R(18),
m représentant 1, 2, 3, 4,
R(2) et R(3), indépendamment l'un de l'autre, sont définis comme R(1), et
R(4) représente un groupe alkyle en C₁-C₃, F, Cl, Br, I, CN, -(CH₂)ₙ-(CF₂)ₒ-CF₃,
n représentant zéro ou 1,
o représentant zéro, 1 ou 2,
le composé o-chlorobenzoylguanidine (chlorhydrate) étant toutefois exclu,
et leurs sels pharmaceutiquement acceptables.

2. Composés de formule I selon la revendication 1, caractérisés en ce que
R(1) représente H, F, Cl, Br, CN, NO₂, un groupe alkyle en C₁-C₈, cycloalkyle en C₃-C₈, Xₐ-(CF₂)_{c}-CF₃,
X représente un atome d'oxygène ou de soufre
a est égal à zéro ou 1,
c est égal à zéro, 1, 2 ou 3,
ou
R(1) représente -SR(10), -OR(10),
R(10) représente un groupe
-C_{f}H_{2f}-cycloalkyle(C₃-C₈), hétéroaryle en C₁-C₉ ou phényle, les systèmes aromatiques n'étant pas substitués ou portant 1 à 3 substituants choisis dans l'ensemble constitué par F, Cl, CF₃, CH₃, les groupes méthoxy, hydroxyle, amino, méthylamino, diméthylamino,
f étant égal à zéro ou 1,
ou
R(1) représente un groupe phényle, naphtyle, biphénylyle, hétéroaryle en C₁-C₉,
ce dernier étant relié par C ou N,
et qui ne sont pas substitués ou portent 1 à 3 substituants choisis dans l'ensemble constitué par F, Cl, CF₃, CH₃, les groupes méthoxy, hydroxyle, amino, méthylamino et diméthylamino,
ou
(R1) représente -SR(13), -OR(13), -NHR(13), -NR(13)R(14), -C≡CR(18), -C[R(19)]=CHR(18),
R(13), R(14) sont identiques ou différents et représentent un groupe
-(CH₂)_{g}-(CHOH)ₕ-(CH₂)ᵢ-(CHOH)ⱼ-R(17),
R(17) étant un atome d'hydrogène ou le groupe méthyle,
-(CH₂)_{g}-O-(CH₂-CH₂O)ₕ-R(24),
g, h, i étant identiques ou différents
et représentant zéro, 1, 2,
j représentant 1, 2,
R(18) représente un groupe phényle
qui est non substitué ou porte 1 à 3 substituants choisis dans l'ensemble constitué par F, Cl, CF₃, les groupes méthyle, méthoxy ou NR(25)R(26), R(25) et R(26) représentant H ou un groupe alkyle en C₁-C₄, ou
R(18) représente un groupe hétéroaryle en C₁-C₉,
qui est non substitué ou substitué comme le groupe phényle, ou
R(18) représente un groupe alkyle en C₁ -C₆,
qui n'est pas substitué ou porte 1 à 3 groupes OH, ou
R(18) représente un groupe cycloalkyle en C₃-C₈, et
R(19) représente un atome d'hydrogène ou le groupe méthyle,
R(2) et R(3), indépendamment l'un de l'autre, sont définis comme R(1), et
R(4) représente un groupe alkyle en C₁-C₂, F, Cl, Br, CN, -(CH₂)ₙ-(CF₂)ₒ-CF₃,
n représentant zéro ou 1,
o représentant zéro ou 1,
et leurs sels pharmaceutiquement acceptables.

3. Composés de formule I selon la revendication 1, caractérisés en ce que
R(1) représente H, F, Cl, un groupe alkyle en C₁-C₈, cycloalkyle en C₅-C₆, Xₐ-(CF₂)_{c}-CF₃,
X représente un atome d'oxygène,
a est égal à zéro ou 1,
c est égal à zéro ou 1,
ou
R(1) représente -SR(10), -OR(10),
R(10) représente un groupe cycloalkyle en C₄-C₆ , quinolyle, isoquinolyle, pyridyle, phényle, qui n'est pas substitué ou porte 1 à 3 substituants choisis dans l'ensemble constitué par F, Cl, CF₃, CH₃, les groupes méthoxy, hydroxyle, amino, méthylamino, diméthylamino,
ou
R(1) représente un groupe phényle, quinolyle, isoquinolyle, pyridyle, imidazoyle,
relié par C ou N,
qui ne sont pas substitués ou portent 1 à 3 substituants choisis dans l'ensemble constitué par F, Cl, CF₃, CH₃, les groupes méthoxy, hydroxyle, amino, méthylamino et diméthylamino,
ou
(R1) représente -C≡CR(18),
R(18) représente un groupe phényle ou cycloalkyle en C₅-C₆,
R(2) et R(3), indépendamment l'un de l'autre, sont définis comme R(1), et
R(4) représente le groupe méthyle, F, Cl, CF₃,
et leurs sels pharmaceutiquement acceptables.

4. Procédé pour la préparation d'un composé I selon la revendication 1, caractérisé en ce que l'on fait réagir avec de la guanidine un composé de formule II 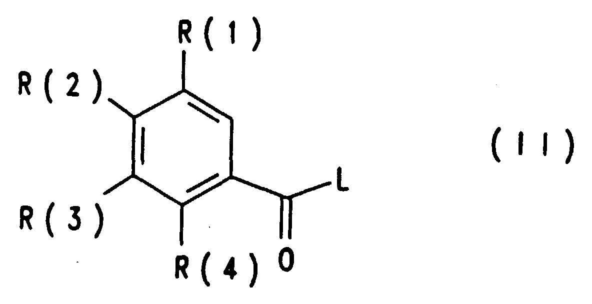 dans laquelle R(1) à R(4) ont les significations données dans la revendication 1 et L représente un groupe partant pouvant être aisément remplacé par substitution nucléophile.

5. Utilisation d'un composé I selon la revendication 1, pour la fabrication d'un médicament destiné au traitement d'arythmies.

6. Utilisation d'un composé I selon la revendication I, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé S selon la revendication 1, pour la fabrication d'un médicament destiné au traitement ou à la prophylaxie d'états ischémiques du système nerveux périphérique et central et de l'apoplexie cérébrale.

10. Utilisation d'un composé S selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d'organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Médicament, caractérisé par une une teneur efficace en un composé de formule I selon la revendication 1.
